# EUROPEAN PATENT SPECIFICATION

(11) **EP 1 536 826 B1**
(45) Date of publication and mention of the grant of the patent: **28.09.2011**
(21) Application number: 03790946.2
(22) Date of filing: 28.08.2003
(51) Int. Cl.: A61P 5/50, A61K 38/30

(54) **IGF-2 PEPTIDE FOR USE IN TOLEROGENIC APPROACHES FOR TYPE I DIABETES.**
IGF-2 PEPTID ZUR VERWENDUNG IN EINER TOLEROGENEN BEHANDLUNG FÜR TYP I DIABETES
IGF-2 PEPTIDE UTILISE DANS DES APPROCHES TOLEROGENES DESTINEES AU DIABETE DE TYPE I.

(30) Priority: 30.08.2002 US 407273 P; 09.12.2002 US 432295 P; 02.01.2003 US 438029 P
(43) Date of publication of application: 08.06.2005
(73) Proprietor: Université de Liège, 4031 Angleur (BE)
(72) Inventor: GEENEN, Vincent, B-4000 LIEGE (BE)
(86) International application number: PCT/EP2003/009623
(87) International publication number: WO 2004/019965

(56) References cited:
- EP-A- 0 266 057
- WO-A-01/72323
- WO-A-91/03253
- US-A- 5 804 417
- GEENEN V ET AL: "ROLE OF THE THYMUS IN THE DEVELOPMENT OF TOLERANCE AND AUTOIMMUNE TOWARDS THE NEUROENDOCRINE SYSTEM" ANNALS OF THE NEW YORK ACADEMY OF SCIENCES, NEW YORK ACADEMY OF SCIENCES, NEW YORK, NY, US, vol. 992, May 2003 (2003-05), pages 186-195, XP008025927 ISSN: 0077-8923
- GEENEN V ET AL: "THYMUS TOLERANCE DYSFUNCTION IN THE DEVELOPMENT OF THE AUTOIMMUNE DIABETOGENIC RESPONSE: A WAY FOR A NOVEL TYPE OF VACCINE/IMMUNOTHERAPY" DIABETOLOGIA, BERLIN, DE, vol. 46, no. SUPPL 2, August 2003 (2003-08), page A10, XP008025930 ISSN: 0012-186X
- KECHA-KAMOUN O ET AL: "THYMIC EXPRESSION OF INSULIN-RELATED GENES IN AN ANIMAL MODEL OF AUTOIMMUNE TYPE 1 DIABETES" DIABETES/METABOLISM RESEARCH AND REVIEWS, WILEY, LONDON,, GB, vol. 17, no. 2, March 2001 (2001-03), pages 146-152, XP008025928 ISSN: 1520-7552

### SEQUENCE LISTING

<110> University de Liege
<120> TOLEROGENIC APPROACH FOR TYPE 1 DIABETES
<130> 2002-17
<150> 60/438029
   <151> 2003-01-02
<150> 60/432295
   <151> 2002-12-09
<150> 60/407273
   <151> 2002-08-30
<160> 3
<170> FastSEQ for Windows Version 4.0
<210> 1
   <211> 67
   <212> PRT
   <213> Homo sapiens
<400> 1
   Ala Tyr Arg Pro Ser Glu Thr Leu Cys Gly Gly Glu Leu Val Asp Thr
    1               5                  10                  15
   Leu Gln Phe Val Cys Gly Asp Arg Gly Phe Tyr Phe Ser Arg Pro Ala
               20                  25                  30
   Ser Arg Val Ser Arg Arg Ser Arg Gly Ile Val Glu Glu Cys Cys Phe
           35                  40                  45
   Arg Ser Cys Asp Leu Ala Leu Leu Glu Thr Tyr Cys Ala Thr Pro Ala
       50                  55                  60
   Lys Ser Glu
   65
<210> 2
   <211> 15
   <212> PRT
   <213> Homo sapiens
<400> 2
   Gly Glu Leu Val Asp Thr Leu Gln Phe Val Cys Gly Asp Arg Gly
    1               5                  10                  15
<210> 3
   <211> 15
   <212> PRT
   <213> Homo sapiens
<400> 3
   Ser His Leu Val Glu Ala Leu Tyr Leu Val Cys Gly Glu Arg Gly
    1               5                  10                  15

## Description

### Background of the Invention

Diabetes Mellitus is the most common metabolic disease worldwide. Every day, approximately 1700 new cases of diabetes are diagnosed in the United States, and at least one third of the 16 million Americans with diabetes are unaware of it. Complications of diabetes impair the longevity and quality of life, and include atherosclerotic heart disease, gangrene and stroke, as well as diabetic retinopathy (retinal change leading to blindness), neuropathy and nephropathy.

Normal glucose homeostasis requires the finely tuned orchestration of insulin secretion by pancreatic beta cells in response to subtle changes in blood glucose levels, delicately balanced with secretion of counter-regulatory hormones such as glucagon. Diabetes can be separated into two types: Type I and Type II. Type I diabetes (or IDDM), which represents 10% of all human diabetes, results from autoimmune destruction of pancreatic beta cells causing insulin deficiency. Type II diabetes or noninsulin-dependent diabetes mellitus (NIDDM) accounts for ±90% of cases and is characterized by a triad of (1) resistance to insulin action on glucose uptake in peripheral tissues, especially skeletal muscle and adipocytes, (2) impaired insulin action to inhibit hepatic glucose production, and (3) dysregulated insulin secretion (See, *e.g*., DeFronzo (1997) Diabetes Rev. 5:177-269). In most cases, type II diabetes is a polygenic disease with complex inheritance patterns (reviewed in Kahn, et al., (1996) Annu. Rev. Med. 47:509-531).

The series of autoimmunologic events which give rise to IDDM, or which are otherwise involved with IDDM onset, are poorly understood. Included in this deleterious autoimmune response are effector immune cells, *i.e.* CD4⁺ and CD8⁺ T lymphocytes, including immunoglobulin-secreting B cells, as well several autoantigens, including Insulin, glutamic acid decarboxylase (GAD) 65 and 67 isotypes, heat shock protein 60, IA-2 and some uncharacterized ICAs (see, *e.g*., Atkinson, M.A. (1994) N. Engl. J. Med. 331:1428-1436; Delovitch, T.L. et al. (1997) Immunity 7:727-738; and Durinovic-Bello, I. (1998) Autoimmunity 27:159-177). Of these identified autoantigens, insulin has been shown to play an important role in the development both humans and animal models of IDDM (see, *e.g*., Semple, J.W. et al. (1992) Int. Immunol. 4:1161-1167; Wegmann, D.R. et al. (1994) J. Autoimmun. 7:833-843; Rudy, G. et al. (1995) Mol. Med. 1:625-633; and Griffin, A.C. et al. (1995) Am. J. Pathol. 147:845-857). However, despite the characterization of these autoantigens, the pathophysiology of the autoimmune process remains unclear.

One current hypothesis is that specific self-reactivity of the immune system may develop from a defect in the thymic establishment of central self tolerance (see, *e.g*., Martens, H. et al. (1996) Immunol. Today 17:312-317; Geenen, V. et al. (1998) Diabetes Metab. Rev. 14:95-103; and Geenen, V. et al. (1998) J. Neuroendocrinol. 10:811-822). Following their formation within the primary hematopoietic sites (*i.e*., fetal yolk sac, fetal liver and then the bone marrow), lymphoid stem cells migrate into the thymus where they are induced by thymic signals to undergo a program of proliferation, gene rearrangement and differentiation. These specific thymic events result in the production of a large number of immature double positive CD4⁺/CD8⁺ thymocytes expressing a diverse repertoire of T-cell antigen receptor (TCR) combinations. This repertoire is then submitted to stringent selection so that T-cells that recognize self-antigens in the context of major histocompatibility complexes (MHC) are induced to die by neglect or by negative selection (see, *e.g*., Kisielow, P. et al. (1988) Nature 333:742-746). This efficient deletion of self-reactive T lymphocytes is responsible for the central self-tolerance of the immune system. The tolerogenic function of the thymus is mediated by the different cellular components of the thymic parenchyme: epithelial/nurse cells (TEC/TNC), macrophages and dendritic cells (see, *e.g*., Bonomo, A. et al. (1993) J. Exp. Med. 177:1153-1164 and Sprent, J. (1995) Int. Rev. Immunol. 13:95-105). Central T cell tolerance of neuroendocrine functions has been proposed to be mediated by the intrathymic expression of one dominant member of the neuroendocrine families (see, *e.g*., Martens, H. *et al.* (1996) *supra* and Geenen, V. *et al.* (1998) *supra*).

Several procedures have been performed in order to restore immunological tolerance of insulin without success. For example, oral administration of insulin failed to protect the residual β cell function in recent-onset IDDM (see, *e.g*., Chaillous, L. et al. (2000) Lancet 356:545-549 and Pozzilli, P. et al. (2000) Diabetologia 43:1000-1004). Moreover, subcutaneous administration of insulin in high-risk relatives of patients with IDDM did not delay or prevent the onset of disease (see, *e.g*., DPT-Type I Diabetes Study Group (2002) N. Engl. J. Med. 346:1685-1691). Another study by Neurocrine Bioscience, Inc. (San Diego, California), demonstrated that a cellular immune response to the sequence B₉₋₂₃ of insulin occurred in patients with IDDM (see, *e.g*., Alleva, D.G. et al. (2001) J. Clin. Invest. 107:173-180). Specifically, the cellular immune response was a CD4-mediated, HLA-II restricted immune response which exhibited a proinflammatory profile with a high production of IFN-γ in response to the presentation of the insulin B₉₋₂₃ peptide. In each of the above trials, use of the insulin autoantigen, or peptides thereof, failed to result in the prevention or treatment of IDDM. Moreover, the potent immunogenic properties of the sequence B₉₋₂₃ of insulin were further recently confirmed since peptide B₉₋₂₃ accelerates incidence of diabetes and induces fatal anaphylaxis in NOD mice (see, *e.g*., Liu, E. et al. (2002) J. Clin. Invest. 110: 1021-1027). There thus remains a need for a treatment that will improve the body's ability to induce tolerance against autoantigens implicated in the destruction of pancreatic β cells.

### Summary of the Invention

The present invention provides an effective method of inhibiting an autoimmune response, *e.g*., a Th₁-mediated immune response, for the treatment and/or prevention of IDDM. The present invention is based, at least in part, on the finding that neuroendocrine self-antigens exert strong tolerogenic or regulatory effects on the immune system as opposed to homologous (or 'altered' self) neuroendocrine autoantigens. Thus, while insulin is the major and most β cell -specific autoantigen of type 1 diabetes, Insulin-Growth Factor (IGF)-2 is the tolerogenic self-antigen precursor of the insulin family. The insulin gene/protein family consists primarily of three members in mammals, insulin (INS), insulin-like growth factor 1 (IGF-1) and insulin-like growth factor 2 (IGF-2). At the protein level, IGF-2 has been identified as the dominant member of this family, expressed in thymic epithelial cells from different species (see, *e.g*., Greenen, V. et al. (1993) Thymus 21:115-127). INS transcripts were also identified in the thymus and the levels of INS mRNA correlated with the alleles of susceptibility to IDDM, however, thymic INS levels are very low (see, *e.g*., Jolicoeur, C. et al. (1994) Proc. Natl. Acad. Sci. USA 91:6707-6711; Pugliese, A. et al. (1997) Nat. Genet. 15:293-297; and Vafiadis, P. et al. (1997) Nat. Genet. 15:289-292).

The invention provides uses in methods of inducing or restoring tolerance in a subject at risk for developing type I diabetes or suffering from type I diabetes, respectively. These include administering to the subject an IGF-2 peptide of the present invention in an amount effective to induce or restore tolerance in the subject.

In another embodiment, the invention provides a vaccine for use in protecting a subject at risk for developing type I diabetes, where the vaccine comprises an IGF-2 peptide of the present invention and a pharmaceutically acceptable carrier. In another aspect, the invention provides a vaccine for use in inducing tolerance in a subject at risk for developing type I diabetes, where the vaccine includes an IGF-2 peptide of the present invention and a pharmaceutically acceptable carrier. In yet another aspect, the invention provides a pharmaceutical composition for use comprising an IGF-2 peptide of the present invention and a pharmaceutically acceptable carrier.

The IGF-2 peptide of the present invention includes the IGF-2 protein of SEQ ID NO: or fragment thereof, capable of modulating an immune response. In certain embodiments, the IGF-2 peptide comprises at least 5, 10, 15, 20, 25, 30, 35, 40, 45, 50, 55, 60, 65, 70, 75, 80, 85, 90, 95 or 100 amino acids. In another embodiment, the IGF-2 peptide of the present invention comprises the amino acid sequence GELVDTLQFVCGDR (SEQ ID NO:2; B₁₁₋₂₅). In another embodiment, the IGF-2 peptide is an altered peptide ligand (APL). In certain embodiments, the IGF-2 peptide of the present invention is capable of binding to the HLA-D allele of a subject. In one embodiment, the HLA-D allele is HLA-DQ. In another embodiment, the HLA-D allele is HLA-DQ8. In another embodiment, the HLA-DQ allele is HLA-DQ2.

In certain embodiments, the IGF-2 peptide is modified, *e.g*., altered or varied, in order to increase the stability of the IGF-2 peptide. In another embodiment, the modification of the IGF-2 peptide further decreases the low immunogenicity of the IGF-2 peptide. In certain embodiments, the IGF-2 peptide is modified by PEGylation. In other embodiments, the IGF-2 peptide is modified by complexing the IGF-2 peptide with a MHC class II dimer.

### Brief Description of the Drawings

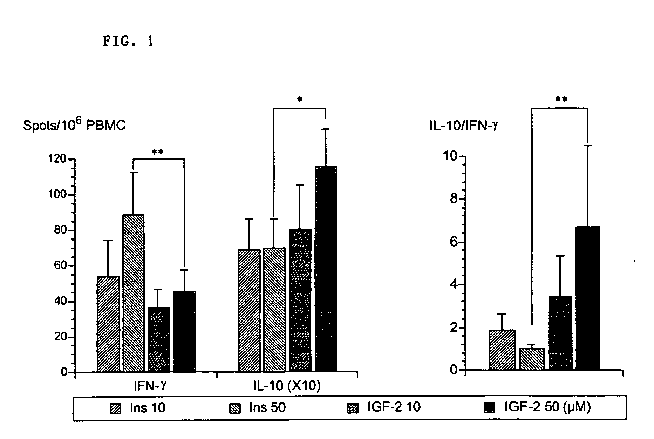

*Figure 1*: ELISPOT^{®} analyses of IFN-γ and IL-10 production in response to insulin peptide B₉₋₂₃ (INS) and IGF-2 peptide B₁₁₋₂₅ in 10 Type 1 diabetic DQ8⁺ adolescents.

### Detailed Description of the Invention

### Definitions

Before further description of the present invention, and in order that the invention may be more readily understood, certain terms are first defined and collected here for convenience.

As used in the present specification, the term "IGF-2 peptide" or "IGF-2 molecule" refers to naturally-occurring IGF-2, natural and unnatural functional analogs and fragments and derivatives thereof capable of inhibiting an immune response. The amino acid sequence of IGF-2 is set forth as (SEQ ID NO: 1). A multitude of protected, unprotected, and partially protected natural and unnatural functional analogs and derivatives of IGF-2 are also intended to be within the scope of the invention. As known in the art, amino acid residues may be in their (1) protected form in which both amino and carboxy groups possess appropriate protecting groups, (2) partially-protected form in which either amino or carboxy groups possess appropriate protecting groups, or (3) unprotected form in which neither amino nor carboxy groups possess an appropriate protecting group. Numerous reactions for the formation and removal of such protecting groups are described in a number of standard works including, for example, "Protective Groups in Organic Chemistry", Plenum Press (London and New York, 1973); Green, T. H., "Protective Groups in Organic Synthesis", Wiley (New York, 1981); and "The Peptides", Vol. I, Schroder and Lubke, Academic Press (London and New York, 1965). All such forms are encompassed within the present invention. Representative amino protecting groups include, for example, formyl, acetyl, isopropyl, butoxycarbonyl, fluorenylmethoxycarbonyl, carbobenzyloxy, and the like. Representative carboxy protecting groups include, for example, benzyl ester, methyl ester, ethyl ester, t-butyl ester, p-nitro phenyl ester, and the like.

In addition to protected forms in which both amino and carboxy groups possess appropriate protecting groups, the IGF-2 peptide of the present invention may also be modified. As used herein, the term "modified" refers to those IGF-2 peptides which have been modified, *e.g*., altered, varied and the like, in which to increase the stability or half-life of the IGF-2 peptide. Such modification include, but are not limited to, PEGylation, complexing the IGF-2 peptide with an MHC tetramer, *e.g*., an MHC class II tetramer, and the like. Processes for preparing these modified peptides are well known to those of ordinary skill.

Also within the scope of the invention are derivatives of naturally-occurring IGF-2 peptides which are obtained by fragmenting a naturally-occurring sequence, or are synthesized based upon a knowledge of the sequence of the naturally-occurring amino acid sequence of the genetic material (DNA or RNA) which encodes the sequence. As used herein, the term "altered peptide ligand" refers to chemical modifications of natural or unnatural IGF-2 peptides and analogs of IGF-2 peptides in which one or more amino acids which are not present in the original sequence are added or deleted, and derivatives thereof. Examples of altered peptide ligands include, but are not limited to, those described by Alleva, D.G. *et al.* (see, *e.g*., Alleva, D.G. et al. (2002) Diabetes 51:2126-2134) wherein the B₉₋₂₃ peptide of INS was modified by inserting alanine substitutions at various places within the peptide, the altered peptide NBI-6024, as described US Patent No. 6,197,926, which includes alanine substitutions at positions 16 and 19, and those described by Kappos, *et al.* (see, *e.g*., Kappos et al., (2000) Nat. Med. 1176-1182) which include the altered mylein basic protein peptide D-Ala83-Lys84-Ley89-Ala91-MBP₈₃₋₉₉.

It will be appreciated that a portion or segment of the described sequence can also be used in the invention so long as it is sufficiently characteristic of the desired IGF-2 peptide or fragment thereof to cause an effect on the immune system which regulates T cells recognizing self-antigens (*i.e*. autoimmunogenic T cells specific for pancreatic islet β cells), but not against T cells not recognizing self-antigens. Such variations in the sequence can easily be made, for example by synthesizing an alternative sequence. The alternate sequence can then be tested, for example, in a model such as a vertebrate model, to ascertain its effectiveness.

As used herein, the term "fragment" means a subset of the conserved amino acid sequence of an IGF-2 peptide that can cause an effect (*e.g*., induce tolerance, inhibit a Th₁ autoimmune response, induce a Th₂-mediated immune response and the like) on the immune system which regulates T cells. The term is intended to include such fragments in conjunction with or combined with additional sequences or moieties, as for example where the peptide is coupled to other amino acid sequences or to a carrier. The terms "fragment" and "peptide" can, therefore, be used interchangeably since a peptide will be the most common fragment of the IGF-2 protein. Reference herein to a "fragment," "portion" or "segment" of an IGF-2 peptide does not mean that the composition must be derived from the intact IGF-2 protein. Such "fragments," portions" or "segments" can be produced by various means well-known to those skilled in the art, such as, for example, manual or automatic peptide synthesis, various methods of cloning, or enzymatic treatment of a whole IGF-2 protein. In certain embodiments, the IGF-2 peptide comprises the amino acid sequence GELVDTLQFVCGDR (SEQ ID NO:2). In other embodiments, the IGF-2 peptide comprises at least 5, 10, 15, 20, 25, 30, 35, 40, 45, 50, 55, 60, 65, 70, 75, 80, 85, 90, 95 or 100 amino acids. In certain embodiments, the IGF-2 peptide of the present invention is capable of binding to the HLA-D allele of a subject. In one embodiment, the HLA-D allele is HLA-DQ. In another embodiment, the HLA-D allele is HLA-DQ8. In another embodiment, the HLA-D allele is HLA-DQ2.

The term "peptide" or "polypeptide" is used in its broadest sense, *i.e*., any polymer of amino acids (dipeptide or greater) linked through peptide bonds. Thus, the term "peptide" includes proteins, oligopeptides, protein fragments, muteins, fusion proteins and the like. The term "protein" is used herein to designate a naturally occurring polypeptide. Peptides of the present invention can be made synthetically, using techniques that are known in the art, or encoded by a nucleic acid, such as DNA or RNA.

The present invention also includes a recombinant molecule comprising a nucleic acid sequence encoding an IGF-2 peptide, operatively linked to a vector capable of being expressed in a host cell. A DNA "coding sequence" or a "nucleotide sequence encoding" a particular peptide or protein, is a DNA sequence which is transcribed and translated into a polypeptide in a host cell when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, prokaryotic sequences, cDNA sequences, cDNA from eukaryotic mRNA, genomic DNA sequences from eukaryotic (*e.g*., mammalian) DNA, and even synthetic DNA sequences. A transcription termination sequence will usually be located 3' to the coding sequence.

As used herein, "operatively linked" refers to insertion of a nucleic acid sequence into an expression vector in such a manner that the sequence is capable of being expressed when transformed into a host cell. As used herein, an "expression vector" is an RNA or DNA vector capable of transforming a host cell and effecting expression of an appropriate nucleic acid sequence, *e.g*., replicating within the host cell. An expression vector can be either prokaryotic or eukaryotic, and typically is a virus or a plasmid.
Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e.g*., DNA) into a host cell, including calcium phosphate or calcium chloride co-precipitation, DEAE-dextran-mediated transfection, lipofection, or electroporation. Suitable methods for transforming or transfecting host cells can be found in Sambrook et al. (Molecular Cloning: A Laboratory Manual. 2nd ed., Cold Spring Harbor Laboratory, Cold Spring Harbor Laboratory Press, Cold Spring Harbor, NY, 1989), and other laboratory manuals.
For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g*., resistance to antibiotics) is generally introduced into the host cells along with the gene of interest. Preferred selectable markers include those which confer resistance to drugs, such as G418, hygromycin and methotrexate. Nucleic acid encoding a selectable marker can be introduced into a host cell on the same vector as that encoding an IGF-2 peptide of the present invention or can be introduced on a separate vector. Cells stably transfected with the introduced nucleic acid can be identified by drug selection (*e.g*., cells that have incorporated the selectable marker gene will survive, while the other cells die).

The regulatory sequences include those required for transcription and translation of the nucleic acid, and may include promoters, enhancers, polyadenylation signals and sequences necessary for transport of the molecule to the appropriate cellular compartment. When the nucleic acid is a cDNA a recombinant expression vector, the regulatory functions responsible for transcription and/or translation of the cDNA are often provided by viral sequences. Examples of commonly used viral promoters include those derived from polyoma, Adenovirus 2, cytomegalovirus and Simian Virus 40, and retroviral LTRs.

Regulatory sequences linked to the nucleic acid can be selected to provide constitutive or inducible transcription, by, for example, use of an inducible enhancer. Thus, in a specific embodiment of the invention the nucleic acid molecule, which encodes for an IGF-2 peptide of the present invention, is under control of an inducible control element, such that expression of the gene can be turned on or off, by contacting or not contacting, respectively, the host cell(s) containing the nucleic acid with an agent which affects the inducible control element. In other embodiments of the invention, the nucleic acid molecule, which encodes for an IGF-2 peptide of the present invention, is under the control of a promoter which constitutively drives the expression of the nucleic acid molecule. Regulatory elements which drive constitutive expression of nucleic acid molecules to which they are operably linked can be viral elements (*e.g*., derived from polyoma, Adenovirus 2, cytomegalovirus, Simian Virus 40 or retrovirus).

Suitable host cells can be any cells that are capable of producing the peptides of the present invention. Such host cells include, but are not limited to, bacterial, fungal, insect and mammalian cells. Host cells of the present invention can also be cells which naturally express an MHC molecule, or are capable of expressing an MHC molecule, and can produce the peptides of the present invention and present them on a MHC molecule. Examples include, but are not limited to, T cells and antigen presenting cells, such as B cells, dendritic cells, and macrophages. Other examples include non-immune cells which express MHC class I molecules on the cell surface, and include, but are not limited to, fibroblasts, epithelial cells and endothelial cells.

A "promoter sequence" is a DNA regulatory region capable of binding RNA polymerase in a cell and initiation transcription of a downstream (3' direction) coding sequence. For purposes of defining the present invention, the promoter sequence is bound at the 3' terminus by the translation start codon (ATG) of a coding sequence and extends upstream (5' direction) to include the minimum number of bases or elements necessary to initiate transcription at levels detectable above background. Within the promoter sequence will be found a transcription initiation site (conveniently defined by mapping with nuclease S1), as well as protein binding domains (consensus sequences) responsible for the binding of RNA polymerase. Eukaryotic promoters will often, but not always, contain "TATA" boxes and "CAT" boxes. Prokaryotic promoters contain Shine-Dalgarno sequences in addition to the - 10 and -35 consensus sequences.

The term "type I diabetes" or "insulin-dependent diabetes mellitus (IDDM)" (also known as "juvenile onset diabetes" or "ketosis-prone diabetes") refers to the condition characterized by the severe, often absolute lack of insulin caused by the reduction of pancreatic islet P cells mass. Type I diabetes usually develops in childhood, becoming manifest and severe at puberty. Patients suffering from type I diabetes depend on insulin for survival; *i.e*. without insulin, patients develop acute metabolic complications, such as acute ketoacidosis and coma.

Uses of the present invention are useful for inducing or restoring tolerance in a subject at risk for or suffering from type I diabetes. The present invention is directed to the treatment and/or prevention of initial onset or recurrent Type I diabetes mellitus in subjects exhibiting Type I diabetes or who are "at risk" or at high risk" of developing Type I diabetes, either recurrently, or for the first time. Subjects who are particularly at risk of developing recurrent Type I diabetes are diabetic humans or animals who were previously diabetic but made non-diabetic due to a transplantation of pancreatic islet β cells. Accordingly, a subject "at risk for" or "at high risk for" is one who possess any one or a combination of the following traits as determined by several tests known in the art:
*(1) Positive for the HLA-D allele.* Genetic susceptibility to diabetes has become increasingly definable through the use of molecular biological means, usually from DNA samples obtained from peripheral blood. One major gene involved in the inherited susceptibility to IDD is that located at the HLA-DQ locus, *e.g*., HLA-DQ8 and HLA-DQ2. It is currently possible to identify risks varying from essentially none to those as high as 70 fold above those without the genotype. In certain families, a genetic risk as high as 1 in 4 can be estimated for unaffected siblings just through identification of HLA haplotypes shared with the affected proband.
*(2) Presence of autoantibodies.* Persons who have just developed IDD or are in process of developing IDD have a number of disease-specific autoantibodies in their blood. Such autoantibodies include those to islet cell antigens (ICA), to beta cell specific proteins of 64 kDa, which are now believed to be the lower molecular isoform of glutamic acid decarboxylase (GAD65), to native insulin and proinsulin, to tyrosine phosphatase IA-2, and to a number of more minor determinants such as carboxypeptidase-H and heat shock proteins belonging to the hsp-60 family.
*(3) Presence of insulin autoantibodies.* Insulin autoantibodies (IAA) have been observed in untreated, newly diagnosed IDDM patients (see, *e.g*., Palmer et al. (1983) Science 222:1337-1339) as well as in apparently unaffected relatives of diabetic probands. Whereas autoimmunity to insulin could directly cause beta-cell damage, could interfere with the action of endogenous insulin, or could have both effects, some investigators suggested that IAA reflect the rate of islet cell destruction and thus act merely as reporters of aggressive islet directed autoimmunity (see, *e.g*., Ziegler et al. (1989) Diabetes 38:1320-1325; Vardi (1988) Diabetes Care 11:736-739).

The terms "immunological tolerance" or "tolerance to an antigen" or "immune tolerance" include unresponsiveness to an antigen without the induction of a prolonged generalized immune deficiency. Consequently, according to the invention, a tolerant host is capable of reacting to antigens other than the tolerizing antigen. Tolerance represents an induced depression in the response of a subject that, had it not been subjected to the tolerance-inducing procedure, would be competent to mount an immune response to that antigen.

As used herein, the term "graft rejection" refers to an immune reaction directed against grafted tissues and/or organs from other human donors (allografts) or from other species such as sheep, pigs, or non-human primates (xenografts). Therefore, the method of the invention is useful for preventing an immune reaction to transplanted pancreatic islet β cells from other human donors (allografts) or from other species (xenografts). Also included within this definition is "graft versus host disease" of "GVHD," which is a condition where the graft cells mount an immune response against the host. Therefore, the method of the invention is useful in preventing graft versus host disease in cases of mismatched tissues, *i.e*. pancreatic islet β cells for the treatment of type I diabetes, for example.

The terms "induce" "inhibit," "potentiate," "elevate," "increase," "decrease," or the like denote quantitative differences between two states, and refer to at least statistically significant differences between the two states. For example, "an amount effective to inhibit an immune response" means that the immune response will be at least significantly less in a subject as compared to a subject that is not treated. Immune responses can be measured using a number of techniques that are known in the art, including evaluating cytokine production and/or the proliferative activity of T cells, as measured by enzyme-linked immunosorbant assays (ELISAs), ELISPOT^{®} assays or [³H]-Thymidine incorporation assays, respectively. Such terms are applied herein to, for example, assess the amount of cytokine production.

As used herein, the term "subject" is intended to include all vertebrates, *i.e*., human and non-human animals. The term "non-human animals" of the invention includes, but is not limited to, mammals, rodents, mice and non-mammals, such as non-human primates, sheep, dog horse, cow, chickens, amphibians, reptiles and the like. In certain embodiments, the subject is a mammal, *e.g*., a primate, *e.g*., a human. In other embodiments, human subjects include those at risk for or suffering from, type I diabetes.

The term "treatment" or "treating" as used herein refers to either (1) the prevention of a disease or disorder (prophylaxis), or (2) the reduction or elimination of symptoms of the disease or disorder (therapy).

The terms "prevention," "prevent," or "preventing" as used herein refers to inhibiting, averting or obviating the onset or progression of a disease or disorder (prophylaxis).

As used herein, the terms "immune" and "immunity" refers to the quality or condition of being able to resist a particular infectious disease.

The terms "immunize" and "immunization," as used herein, refer to the act of making a subject (1) not susceptible to a disease or disorder; or (2) less responsive to a disease or disorder; or (3) have an increased degree of resistance to a disease or disorder.

The term "MHC class bearing cell" refers to any cell which expresses an MHC molecule, *i.e*. MHC class I or class II molecule, on the cell surface. In humans, almost all nucleated cells express MHC class I molecules, although the level of expression varies between cell types. Cells of the immune system express abundant MHC class I on their surfaces, while liver cells express relatively low levels. MHC class II molecules are primarily expressed on immune cells, particularly antigen presenting cells (APC), *i.e*., B cells, dendritic cells, monocytes and macrophages. However, many other cell types can be induced to express MHC class II molecules and are also meant to be within the scope of the invention. MHC molecules often have different names between vertebrates. For example, MHC is often referred to as HLA in humans and H-2 in mice. These differences in nomenclature are intended to be within the scope of the present invention.

The term "immune cell" includes cells of the immune system which are capable of expressing, producing or secreting cytokines that regulate an immune response, for example a Type-1 (Th₁) or type-2 (Th₂) immune response. Immune cells of the present invention include human immune cells, such as, but not limited to, macrophages, dendritic cells, T cells, B cells and neutrophils.

As used herein, the term "T cell" or "T lymphocyte" is intended to include all cells within the T cell lineage, including thymocytes, immature T cells, mature T cells (including T cells bearing the surface markers CD4 and/or CD8) and the like, from a mammal (*e.g*., human or mouse). The term "cytokine" is meant to include any one of the group of hormone-like mediators produced by T and B lymphocytes. Representative cytokines include, but are not limited to, Interleukin (IL)-1, IL-2, IL-3, IL-4, IL-5, IL-6, IL-7, IL-8, IL-9, 1L-10, 1L-11, IL-12, IL-13, IL-15, IL-18, Interferon (IFN)-γ, Tumor Necrosis Factor (TNF) α, and Transforming Growth Factor (TGF)-β. An "active" fragment of a cytokine is a fragment of a cytokine that retains activity as determined using standard *in vitro* and *in vivo* assays. For example, assays for determining IL-2 and IFN-γ activity are known in the art (see, *e.g*., Campos, M. (1989) Cell. Immunol. 120:259-269 and Czarnieki, C.W. (1986) J. Interferon Res. 6:29-37). Assays for determining the activity of other cytokines are known as well as commercially available and can readily be conducted by those having ordinary skill in the art.

The term "immune response" includes any response associated with immunity including, but not limited to, increases or decreases in cytokine expression, production or secretion (*e.g*., IL-12, IL-10, TGF-β or TNF-α expression, production or secretion), cytotoxicity, immune cell migration, antibody production and/or immune cellular responses. An immune response as used herein may be modulated, including the upregulation, potentiating, stimulating, enhancing or increasing of the immune response, as defined herein. Alternatively, as used herein, modulation of an immune response also includes the downregulation, inhibition or decreasing of an immune response, as defined herein.

Immune responses in a subject can be further characterized as being either Th₁- or Th₂-mediated immune responses.

A "Th₁-mediated immune response," also referred herein as a "type-1 response" includes a response by CD4⁺ T cells that is characterized by the expression, production or secretion of one or more type-1 cytokines and that is associated with many autoimmune responses and delayed-type hypersensitivity responses. The phrase "type-1 cytokine" or "Th₁-type cytokine" includes a cytokine that is preferentially or exclusively expressed, produced or secreted by a Th₁ cell, that favors development of Th₁ cells and/or that potentiates, enhances, or otherwise mediates autoimmune reactions or delayed-type hypersensitivity reactions. Examples of Th₁-type cytokines include, but are not limited to, GM-CSF, IL-2, IFN-γ, TNF-α, IL-12, IL-15 and IL-18.

Included within a Th₁-mediated immune response is an "autoimmune response." As used herein, the term "autoimmune response" refers to the condition where the immune system attacks the host's own tissue(s). In an autoimmune response or disease, the immune tolerance system of the patient fails to recognize self antigens and, as a consequence of this loss of tolerance, brings the force of the immune system to bear on tissues which express the antigen. Accordingly, the term "autoimmune response" includes any response associated with cytotoxic T cell (CD8⁺ T cell) and helper T cell (CD4⁺ T cell) immunity including, but not limited to, increases or decreases in cytokine expression, production or secretion (*e.g*., IL-2, IL-12, IL-15 or IFN-γ expression, production or secretion), cytotoxicity, immune cell migration, antibody production and/or immune cellular responses. The phrase "inhibiting an autoimmune response" includes down-regulating, inhibiting, or decreasing an immune response, as defined herein. For example, an autoimmune response can be down-regulated, inhibited, or decreased directly by use of an IGF-2 peptide of the present invention.

A "Th₂ immune response", also referred to herein as a "type-2 response" or "Th₂-mediated immune response" refers to a response by CD4⁺ T cells that is characterized by the production of one or more type-2 cytokines and that is associated with humoral or antibody-mediated immunity (*e.g*., efficient B cell, "help" provided by Th₂ cells, for example, leading to enhanced IgG₁ and/or IgE production). The phrase "type-2 cytokine" or "Th₂-type cytokine" includes a cytokine that is preferentially or exclusively expressed, produced or secreted by a Th₂ cell, that favors development of Th₂ cells and/or that potentiates, enhances or otherwise mediates antibody production by B lymphocytes. Examples of Th₂-type cytokines include, but are not limited to, IL-4, IL-5, IL-6, IL-10, and IL-13.

Included within a Th₂-mediated immune response is a antibody-mediated immune response. The term "antibody-mediated immune response" or "Th₂-mediated immune response" includes any response associated with T helper T cell (CD4⁺ T cell) immunity including, but not limited to, increases or decreases in cytokine expression, production or secretion (*e.g*., IL-4, IL-5, IL-6, IL-10 or Il-13 expression, production or secretion), immune cell migration, antibody production and/or immune cellular responses. The phrase "modulating a Th₂-mediated immune response" or "modulation of a Th₂-mediated immune response" includes upregulating, enhancing, stimulating or increasing an immune response, as defined herein.

The phrase "type-1 immunity" includes immunity characterized predominantly by type-1 immune responses (*e.g*., delayed type hypersensitivity, macrophage activation and or cellular cytotoxicity), by expression, production or secretion of at least one type-1 cytokine and/or expression of a type-1 immunity cytokine profile. The phrase "type-2 immunity" includes immunity characterized predominantly by type-2 immune responses (*e.g*., B cell help, IgG₁ and/or IgE production, eosinophil activation, mast cell stimulation and/or macrophage deactivation), by expression, production or secretion of at least one type-2 cytokine and/or expression of a type-2 immunity cytokine profile.

### Embodiments of the Invention

The present invention provides, at least in part, uses in methods for preventing and treating type I diabetes in a subject. The invention is based on the discovery that IGF-2, a self antigen precursor of the insulin family, and fragments thereof, exhibit tolerogenic properties and thus can be used in the treatment and/or prevention of type I diabetes.

In another aspect, the invention provides uses in a method of inducing tolerance in a subject at risk for developing type I diabetes, comprising administering to a subject an IGF-2 peptide of the present invention in an amount effective to induce tolerance in the subject.

In yet another aspect, the invention provides uses in a method of restoring tolerance in a subject suffering from type I diabetes, comprising administering to the subject an effective amount of an IGF-2 peptide of the present invention in an amount effective to restore the tolerance in the individual.

The exogenous IGF-2 peptide of the present invention is a peptide which inhibits a Th₁-mediated immune response. In another embodiment, the IGF-2 peptide of the present invention is a peptide which induces a Th₂-mediated immune response.

A nucleic acid encoding an IGF-2 peptide of the present invention can be introduced into, and expressed in, host cells using methods known in the art. Thus, host cells from a subject having type I diabetes mellitus can be transfected *ex vivo* with a nucleic acid encoding an IGF-2 peptide of the present invention, followed by administration of the host cells to the patient. In another embodiment, the nucleic acid molecule encoding the IGF-2 peptide of the present invention is administered to the patient *in vivo. In vivo* gene therapy protocols are set forth in, *e.g*., Dev et al., 1994, Cancer Treat. Rev. 20:105-115, Ragot, T. et al., 1993, Nature 3:647; Dunckley, M. G. et al., 1993, Hum. Mol. Genet. 2:717-723.

The IGF-2 peptide of the present invention may be inserted into cells, *e.g*., immune antigen-presenting cells, using viral or non-viral vectors which include, but are not limited to, vectors derived from, for example, retroviruses, vaccinia virus, adeno-associated virus, herpes viruses, bovine papilloma virus or non-viral vectors, such as plasmids. In addition, techniques frequently employed by those skilled in the art for introducing DNA into mammalian cells may be utilized. For example, methods including but not limited to, electroporation, DEAE-dektran mediated DNA transfer, DNA guns, liposomes, direct injection, and the like, may be utilized to transfer recombinant vectors into host cells. Alternatively, the DNA may be transferred into cells through conjugation to proteins that are normally targeted to the inside of a cell. For example, the DNA may be conjugated to viral proteins that normally target viral particles into the targeted host cell. Techniques such as those described herein may thus be utilized for the introduction of an IGF-2 peptide of the present invention into human cells.

### Therapeutic Methods

The present invention includes administering to a subject having of at risk of developping type I diabetes an effective amount of an IGF-2 peptide of the present invention, thereby inducing or restoring tolerance in a subject at risk for developping or having type I diabetes.

Also within the scope of this invention is the administration of an IGF-2 peptide prophylactically. Administration of an IGF-2 peptide of the present invention can occur prior to the manifestation of symptoms of type I diabetes, such that the type I diabetes is prevented, or alternatively, delayed in its progression. The prophylactic uses of the present invention can be carried out in a similar manner to the therapeutic methods described herein, although dosage and treatment regimens may differ.

Accordingly, the present use has therapeutic utility in modulating an immune response. In certain embodiments, the present method has therapeutic utility in biasing an immune response towards a Th₂-mediated immune response (*e.g*., stimulating the production of Th₂-mediated cytokines) depending upon administration the desired therapeutic regimen. In another embodiment, the present invention has therapeutic utility in inhibiting a Th₁-mediated immune responses (*e.g*., inhibit Th₁ cells from producing Th₁-mediated cytokines). Such methods are useful in treating type I diabetes.

In another aspect, the invention provides a vaccine for immunizing a subject against type I diabetes, wherein the vaccine comprises an IGF-2 peptide of the present invention, either alone or dispersed in a physiologically acceptable, nontoxic vehicle in an amount is effective to immunize a subject against type I diabetes.

The vaccines of the present invention are administered in a manner compatible with the dosage formulation, and in such amount as will be therapeutically effective and tolerogenic. The quantity to be administered depends on the subject be treated, capacity of the subject's immune system to generate a cellular immune response, and degree of protection desired. Precise amounts of active ingredient required to be administered depend on the judgement of the practitioner and are peculiar to each individual. However, suitable dosage ranges are of the order of about 30 micrograms active ingredient per kilogram per 70 kilogram individual. Suitable regimes for initial administration and booster shots are also variable, but are typified by an initial administration followed in one or two week intervals by a subsequent injection or other administration. Also within the scope of the invention is the co-administration of an adjuvant in combination with an IGF-2 peptide of the present invention.

### Pharmaceutical Compositions and Uses Thereof

Also provided are pharmaceutically-acceptable compositions which comprise an IGF-2 peptide of the present invention and a pharmaceutically-acceptable carrier(s), in an amount effective to modulate a CTL-mediated immune response.

The IGF-2 peptide is administered to the subject using a pharmaceutically-acceptable formulation, *e.g*., a pharmaceutically-acceptable formulation that provides sustained delivery of the IGF-2 peptide of the present invention to a subject for at least 12 hours, 24 hours, 36 hours, 48 hours, one week, two weeks, three weeks, or four weeks after the pharmaceutically-acceptable formulation is administered to the subject.

These pharmaceutical compositions are suitable for oral administration to a subject. In other embodiments, as described in detail below, the pharmaceutical compositions may be specially formulated for administration in solid or liquid form, including those adapted for the following: (1) oral administration, for example, drenches (aqueous or non-aqueous solutions or suspensions), tablets, boluses, powders, granules, pastes; (2) parenteral administration, for example, by subcutaneous, intramuscular or intravenous injection as, for example, a sterile solution or suspension; (3) topical application, for example, as a cream, ointment or spray applied to the skin; (4) intravaginally or intrarectally, for example, as a pessary, cream or foam; or (5) aerosol, for example, as an aqueous aerosol, liposomal preparation or solid particles containing the compound.

As used herein, the term "effective amount" includes an amount effective, at dosages and for periods of time necessary, to achieve the desired result, *e.g*., sufficient to modulate a CD4⁺ Th₁ and CTL-mediated immune response. An effective amount of IGF-2 peptide as defined herein may vary according to factors such as the disease state, age, and weight of the subject, and the ability of the IGF-2 peptide of the present invention to elicit a desired response in the subject. Dosage regimens may be adjusted to provide the optimum therapeutic response. An effective amount is also one in which any toxic or detrimental effects (*e.g*., side effects) of the IGF-2 peptide of the present invention of the present invention are outweighed by the therapeutically beneficial effects.

A therapeutically effective amount of IGF-2 peptide (*i.e*., an effective dosage) may range from about 0.001 to 40 µg/kg body weight, preferably about 0.01 to 30 µg/kg body weight per 70 kilogram individual. The skilled artisan will appreciate that certain factors may influence the dosage required to effectively treat a subject, including but not limited to the severity of the disease or disorder, previous treatments, the general health and/or age of the subject, and other diseases present. Moreover, treatment of a subject with a therapeutically effective amount of an IGF-2 peptide can include a single treatment or, can include a series of treatments. In one example, a subject is treated with an IGF-2 peptide in the range of between about 0.1 to 30 µg/kg body weight, one time per week for between about 1 to 10 weeks, preferably between 2 to 8 weeks, more preferably between about 3 to 7 weeks, and even more preferably for about 4, 5, or 6 weeks. It will also be appreciated that the effective dosage of an IGF-2 peptide used for treatment may increase or decrease over the course of a particular treatment.

A therapeutically effective amount of an IGF-2 peptide may be administered in combination with another pharmaceutically active compound known to modulate immune responses, *e.g*., agents such as interleukins (IL) (*e.g.* IL-2, IL-12, IL-15), lipopolysaccharide (LPS), Conconavalin A (ConA), phorbol esters, and ionomycin. Other pharmaceutically active compounds that may be used can be found in Harrison's Principles of Internal Medicine, Thirteenth Edition, Eds. T.R. Harrison et al. McGraw-Hill N.Y., NY; and the Physicians Desk Reference 50th Edition 1997, Oradell New Jersey, Medical Economics Co. The IGF-2 peptide and the pharmaceutically active compound may be administered to the subject in the same pharmaceutical composition or in different pharmaceutical compositions (at the same time or at different times).

The regimen of administration also can affect what constitutes an effective amount. IGF-2 peptide can be administered to the subject prior to, simultaneously with, or after the administration of the other agent(s). Further, several divided dosages, as well as staggered dosages, can be administered daily or sequentially, or the dose can be proportionally increased or decreased as indicated by the exigencies of the therapeutic situation.

The phrase "pharmaceutically acceptable" is employed herein to refer to those IGF-2 peptides compositions containing such compounds, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, solvent or encapsulating material, involved in carrying or transporting the subject chemical from one organ, or portion of the body, to another organ, or portion of the body. Each carrier must be "acceptable" in the sense of being compatible with the other ingredients of the formulation and not injurious to the subject. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Compositions containing an IGF-2 peptide(s) include those suitable for oral, nasal, topical (including buccal and sublingual), rectal, vaginal, aerosol and/or parenteral administration. The compositions may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred per cent, this amount will range from about 1 per cent to about ninety-nine percent of active ingredient, preferably from about 5 per cent to about 70 per cent, most preferably from about 10 per cent to about 30 per cent.

Methods of preparing these compositions include the step of bringing into association an IGF-2 peptide with the carrier and, optionally, one or more accessory ingredients. In general, the formulations are prepared by uniformly and intimately bringing into association an IGF-2 peptide with liquid carriers, or finely divided solid carriers, or both, and then, if necessary, shaping the product.

Compositions suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of an IGF-2 peptide(s) as an active ingredient. An IGF-2 peptide(s) may also be administered as a bolus, electuary or paste.

In solid dosage forms for oral administration (capsules, tablets, pills, dragees, powders, granules and the like), the active ingredient is mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered active ingredient moistened with an inert liquid diluent.

The tablets, and other solid dosage forms of the pharmaceutical compositions, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the IGF-2 peptide(s) include pharmaceutically-acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active IGF-2 peptide(s) may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Pharmaceutical compositions for rectal or vaginal administration may be presented as a suppository, which may be prepared by mixing one or more IGF-2 peptide(s) of the present invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active agent.

Compositions which are suitable for vaginal administration also include pessaries, tampons, creams, gels, pastes, foams or spray formulations containing such carriers as are known in the art to be appropriate.

Dosage forms for the topical or transdermal administration of an IGF-2 peptide includes powders, sprays, ointments, pastes, creams, lotions, gels, solutions, patches and inhalants. The active IGF-2 peptide(s) may be mixed under sterile conditions with a pharmaceutically-acceptable carrier, and with any preservatives, buffers, or propellants which may be required.

The ointments, pastes, creams and gels may contain, in addition to IGF-2 peptide(s) excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to an IGF-2 peptide(s) excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

The IGF-2 peptide(s) can be alternatively administered by aerosol. This is accomplished by preparing an aqueous aerosol, liposomal preparation or solid particles containing the compound. A nonaqueous (*e.g*., fluorocarbon propellant) suspension could be used. Sonic nebulizers are preferred because they minimize exposing the agent to shear, which can result in degradation of the compound.

Ordinarily, an aqueous aerosol is made by formulating an aqueous solution or suspension of the agent together with conventional pharmaceutically-acceptable carriers and stabilizers. The carriers and stabilizers vary with the requirements of the particular compound, but typically include nonionic surfactants (Tweens, Pluronics, or polyethylene glycol), innocuous proteins like serum albumin, sorbitan esters, oleic acid, lecithin, amino acids such as glycine, buffers, salts, sugars or sugar alcohols. Aerosols generally are prepared from isotonic solutions.

Transdermal patches have the added advantage of providing controlled delivery of an IGF-2 peptide(s) to the body. Such dosage forms can be made by dissolving or dispersing the agent in the proper medium. Absorption enhancers can also be used to increase the flux of the active ingredient across the skin. The rate of such flux can be controlled by either providing a rate controlling membrane or dispersing the active ingredient in a polymer matrix or gel.

Ophthalmic formulations, eye ointments, powders, solutions and the like, are also contemplated.

Pharmaceutical compositions suitable for parenteral administration comprise one or more IGF-2 peptide(s) in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of IGF-2 peptide(s) in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

When the IGF-2 peptide(s) are administered as pharmaceuticals, to humans and animals, they can be given per se or as a pharmaceutical composition containing, for example, 0.1 to 99.5% (more preferably, 0.5 to 90%) of active ingredient in combination with a pharmaceutically-acceptable carrier.

The term "administration" or "administering" is intended to include routes of introducing the IGF-2 peptide(s) to a subject to perform their intended function. Examples of routes of administration which can be used include injection (subcutaneous, intravenous, parenterally, intraperitoneally, intrathecal), oral, inhalation, rectal and transdermal. The pharmaceutical preparations are, of course, given by forms suitable for each administration route. For example, these preparations are administered in tablets or capsule form, by injection, inhalation, eye lotion, ointment, suppository, etc. administration by injection, infusion or inhalation; topical by lotion or ointment; and rectal by suppositories. Oral administration is preferred. The injection can be bolus or can be continuous infusion. Depending on the route of administration, the IGF-2 peptide(s) can be coated with or disposed in a selected material to protect it from natural conditions which may detrimentally effect its ability to perform its intended function. The IGF-2 peptide(s) can be administered alone, or in conjunction with either another agent as described above or with a pharmaceutically-acceptable carrier, or both. The IGF-2 peptide(s) can be administered prior to the administration of the other agent, simultaneously with the agent, or after the administration of the agent. Furthermore, the IGF-2 peptide(s) can also be administered in a proform which is converted into its active metabolite, or more active metabolite *in vivo.*

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion.

The phrases "systemic administration," "administered systemically", "peripheral administration" and "administered peripherally" as used herein mean the administration of an IGF-2 peptide(s) drug or other material, such that it enters the subject's system and, thus, is subject to metabolism and other like processes, for example, subcutaneous administration.

Regardless of the route of administration selected, the IGF-2 peptide(s), which may be used in a suitable hydrated form, and/or the pharmaceutical compositions, are formulated into pharmaceutically-acceptable dosage forms by conventional methods known to those of skill in the art.

The invention is further illustrated by the following examples.

### EXAMPLES

This experiment assessed the ability of the IGF-2 peptide B₁₁₋₂₅ to function as a tolerogenic vaccine against type I diabetes.

### Materials and Methods:

A preclinical study was initiated to compare the CD4 responses elicited by DR8 presentation of the sequence B₉₋₂₃ from insulin and the homologous sequence B₁₁₋₂₅ from IGF-2.

The sequences B₉₋₂₃ from insulin and B₁₁₋₂₅ from IGF-2 were synthesized by and purchased from Neosystem (Strasburgh). The sequences from these peptides are as follows:
IGF-2 (B 11-25) (SEQ ID NO:2)* :
GELVDTLQFVCGDRG
Insulin B9-23 (SEQ ID NO:3):
SHLVEALYLVCGERG
* Underscore denotes conserved sequence

Ten diabetic patients were selected (6 females, 4 males; age range: 7-16 years of age) which were positive for the DQ8 allele. With the assistance of endocrine pediatricians, appropriate arrangements were made for two sampling sessions. Written and informed consent was obtained from the parents of the adolescents. For each patient, 20 ml of blood was collected in tubes containing EDTA. These tubes were immediately sent to Cypro SA (Brussels) where peripheral blood mononuclear cells (PBMCs) were isolated using the Lymphopren™ (Invitrogen) isolation technique.

*ELISPOT Assays* (Cypro): The general methodology has been described by Tary-Lehmann *et al.* (see, *e.g*., Tary-Lahmann et al. (1998) Transplantation 66:219-224). Briefly, PBMCs were seeded at a concentration of 1 x 10⁵/well in 96-well plates coated with anti-human IFN-γ monoclonal antibody (mAb) (Serpime/Nodia) or anti-IL-10 mAb (BD-Pharmingen) in the presence or absence of either PHA (Sigma, 1 µg/ml) + LPS (Sigma, 1 µg/ml), Purified Protein Derivative (PPD, 5 µg/ml), Insulin B₉₋₂₃ (10 and 50 µM), IGF-2 B₁₁₋₂₅ (10 and 50 µM). After 48 hours of incubation at 37°C, 5% CO₂ on a plane surface, wells were washed three times with PBS Tween 0.25%, then blot dried.

Cytokines were revealed with the use of anti-human IFN-γ (Serpime/Nodia) and IL-10 (BD-Pharmingen) secondary biotinylated-mAb plus solution of extravidinperoxydase (4 µg/ml). After 1 hour-incubation in a humid room at ambient temperature, the plates were washed and the substrate AEC (3-amino-9-ethylcarazole) + 30% H₂0₂ were added. After 15 minutes (for IFN-γ) or 30 minutes (for IL-10) of incubation at ambient temperature, spots appeared and the reaction was stopped by washing the plates under water. After drying, the spots were counted by image analyzer assisted by computer with filters specific for each cytokine.

A Wilcoxon test was used for statistical analyses (non-parametric paired values).

### Results

As shown Figure 1, the comparison of CD4 responses in DQ8+ type 1 diabetic adolescents after presentation of an IGF-2 peptide of the present invention (50 µM) showed statistically significant differences. Specifically, the results showed:
(1) The number of IFN-γ secreting cells was significantly lower after treatment with IGF-2 B₁₁₋₂₅ than after treatment with insulin B₉₋₂₃ (50 µM) (88.90 ± 23.82 vs. 45.60 ± 11.67 SEM, p = 0.002).
(2) The number of IL-10 secreting cells was significantly higher after treatment with IGF-2 B₁₁₋₂₅ than after treatment with insulin B₉₋₂₃ (50 µM) (1162 ± 208.6 vs. 701.8 ± 160.9 SEM, p = <0.05)
(3) The ratio of IL-10 / IFN-γ was significantly higher after treatment with IGF-2 B₁₁₋₂₅ than after treatment with insulin B₉₋₂₃ (50 µM) (67.36 ± 37.58 vs. 10.22 ± 1.99, p = 0.002)

The results showed that, while there is not change in the presentation by the MHC DQ8 allele, the CD4 response elicited by insulin B₉₋₂₃ and IGF-2 B₁₁₋₂₅ are significantly different and quite opposite. The IFN-γ response to insulin B₉₋₂₃ was expected and correlated with previous observations (see, *e.g*., Alleva *et al.* (2001), *supra*). The production of IL-10 under these conditions was of particular interest. Although insulin B₉₋₂₃ was able to induce IL-10 production, the production was significantly higher in response to IGF-2 B₁₁₋₂₅ at 50 µM. Since the number of IFN-γ secreting cells was lower after IGF-2 B₁₁₋₂₅, the ratio of IL-10 to IFN-γ (IL-10/IFN-γ) was almost 7-fold higher after treatment with IGF-2 B₁₁₋₂₅.

### Conclusion

The results of this experiment suggest two possible hypotheses:
(1) The peripheral T cell repertoire of diabetic patients contains only T cells against insulin-derived epitopes and IGF-2 B₁₁₋₂₅ could be recognized by those cells as a natural 'altered peptide ligand' (APL) eliciting a different response because of a different transduction after binding to TCR specific of DQ8-insulin B₉₋₂₃ complex; or
(2) IGF-2 B₁₁₋₂₅ could stimulate specific CD4⁺CD25⁺ T_{REG} cells specific for IGF-2.

In either case, the data clearly shows that IGF-2 B₁₁₋₂₅ is a potent inducer of the secretion of IL-10, a major regulatory cytokine with immunosuppressive and antiinflammatory properties. Furthermore, the data further supports that the IGF-2 B₁₁₋₂₅ exhibits tolerogenic properties thus possibly explaining why it is difficult to obtain antibodies against IGF-2 after active immunization with this peptide.
1. SEQ ID NO: (Human IGF-2 full length; amino acid):
2. SEQ ID NO:2 (IGF-2 B₁₁₋₂₅; human; amino acid):
   GELVDTLQFVCGDRG (single letter code)
SEQ ID NO:3 (Insulin B₉₋₂₃; human; amino acid):
   SHLVEALYLVCGERG (single letter code)

## Claims

1. An IGF-2 peptide for use in a method of inducing or restoring tolerance in a subject at risk for developing type I diabetes or suffering from type I diabetes.

2. The IGF-2 peptide for use according to claim 1, which comprises the amino sequence GELVDTLQFVCGDRG (SEQ ID NO:2; B₁₁₋₂₅).

3. A vaccine composition for use in protecting a subject at risk for type I diabetes comprising an IGF-2 peptide and a pharmaceutically acceptable carrier therefor, wherein said IGF-2 peptide is in an amount effective to prevent type I diabetes in the subject

4. A vaccine composition for inducing tolerance in a subject at risk for developing type I diabetes comprising an IGF-2 peptide and a pharmaceutically acceptable carrier, wherein the IGF-2 peptide is in an amount effective to induce tolerance in the subject

5. The composition for use according to any one of claim 3 or 4, wherein said IGF-2 peptide comprises the amino sequence GELVDTLQFVCGDRG (SEQ ID NO:2; B₁₁₋₂₅).

## Patentansprüche

1. Ein IGF-2-Peptid zur Verwerdung in einem Verfahren zur Induktion oder Wiederherstellung von Toleranz in einem Patienten, bei dem ein Typ-I-Diabetes-Risiko besteht oder der an Typ-I-Diabetes leidet.

2. Das IGF-2-Peptid zur Verwendung nach Anspruch 1, welches die Aminosäuresequenz GELVDTLQFVCGDRG (SEQ ID NO:2; B₁₁₋₂₅) umfasst.

3. Eine Impfstoffzusammensetzung zur Verwerdung beim Schütz eines Patienten mit Typ-I-Diabetes-Risiko, umfassend ein IGF-2-Peptid und einen hierfür pharmazeutisch unbedenklichen Träger , wobei das IGF-2-Peptid in einer Menge vorliegt, die Typ-I-Diabetes bei dem Patienten wirksam verhindert.

4. Eine Impfstoffzusammensetzung zur Induktion von Toleranz bei einem Patienten, bei dem das Risiko des Auftretens von Typ-I-Diabetes besteht, umfassend ein IGF-2-Peptid und einen pharmazeutisch unbedenklichen Träger, wobei das IGF-2-Peptid in einer Menge vorliegt, die Toleranz in dem Patienten wirksam induziert.

5. Die Zusammensetzung zur Verwendung nach Anspruch 3 oder 4, wobei das IGF-2-Peptid die Aminosäuresequenz GELVDTLQFVCGDRG (SEQ ID NO:2; B₁₁₋₂₅) umfasst.

## Revendications

1. Un peptide IGF-2 pour utilisation dans un procédé pour induire ou restaurer la tolérance chez un sujet à risque de développer le diabète de type I ou souffrant de diabète de type I.

2. Le peptide IGF-2 pour utilisation selon la revendication 1, qui comprend la séquence d'acides aminés GELVDTLQFVCGDRG (SEQ ID NO:2 ; B₁₁₋₂₅).

3. Une composition de vaccin pour utilisation dans la protection d'un sujet à risque de développer le diabète de type I comprenant un peptide IGF-2 et un véhicule pharmaceutiquement acceptable pour celui-ci, où ledit peptide IGF-2 est en une quantité efficace pour prévenir le diabète de type I chez le sujet.

4. Une composition de vaccin pour induire la tolérance chez un sujet à risque de développer le diabète de type I comprenant un peptide IGF-2 et un véhicule pharmaceutiquement acceptable, où le peptide IGF-2 est en une quantité efficace pour induire la tolérance chez le sujet.

5. La composition pour utilisation selon l'une quelconque des revendications 3 ou 4, dans laquelle ledit peptide IGF-2 comprend la séquence d'acides aminés GELVDTLQFVCGDRG (SEQ ID NO:2 ; B₁₁₋₂₅)
